# EUROPEAN PATENT APPLICATION

(11) **EP 2 730 222 A1**
(43) Date of publication of application: **14.05.2014**
(21) Application number: 12192415.3
(22) Date of filing: 13.11.2012
(51) Int. Cl.: A61B 5/1455, A61B 5/024

(54) **Method of measuring a physiological parameter of an organism, measurement system and physiology monitoring device**

(71) Applicant: Nederlandse Organisatie voor Toegepast -Natuurwetenschappelijk Onderzoek TNO, 2628 VK Delft (NL)
(72) Inventor: Dunias, Paraskevas, 2628 VK Delft (NL); Kalisvaart, Sytze H., 2628 VK Delft (NL)
(74) Representative: Jansen, Cornelis Marinus

(57) **Abstract**

The present invention is directed at a method of and system for measuring a parameter of a body, in particular a physiologic parameter of an organism, such as a human or animal body, or a concentration of a substance in a contained volume. The method is performed using a measurement system comprising an illumination unit and a sensor unit. The method comprising illuminating a body part of the body by providing electromagnetic radiation to an illumination area on the body part using the illumination unit, receiving by the sensor unit at least part of said radiation which has travelled via the body part, and analysing by a processing unit an output signal from the sensor unit using an analysis algorithm for determining a measurement value of said physiological parameter. Illumination of the body part is performed by emitting the radiation from a planar radiation emitting surface of the illumination unit for providing the radiation comprising a substantially planar homogeneous wave front in at least a part of the illumination area.

## Description

The present invention is directed at a method of measuring a physiologic parameter of an organism, such as a human or animal body, or a concentration of a substance in a contained volume, wherein said method is performed using a measurement system comprising an illumination unit and a sensor unit, the method comprising illuminating a body part of the body by providing electromagnetic radiation to an illumination area on the body part using the illumination unit, receiving by the sensor unit at least part of said radiation which has travelled via the body part, and analyzing by a processing unit an output signal from the sensor unit using an analysis algorithm for determining a measurement value of said physiological parameter.

The present invention further relates to measurement system for measuring a physiologic parameter of an organism such as a human or animal body, using a method as described above. In accordance with a further aspect, the present invention is directed at a physiologic monitoring device containing such a measurement system.

### Background

Optical physiologic measurement systems and devices, such as oximeters and similar measurement systems, are known for enabling the non invasive measurement of blood related values. Such measurement systems may be directed at measuring various blood values such as the oxygen level, glucose level, the saturation of a persons oxy-hemoglobin and de-oxyhemoglobin, and other parameters.

In most cases, a body part such as a finger of a patient is held in between an illumination unit and an optical sensor. Alternatively, the illumination unit and sensor operate in a reflective mode, whereby the sensor and the illumination unit may be located at a same side of the body part. In a reflective mode, the sensor receives a reflected part of the radiation illuminated by the illumination unit into the body part. In both the transmissive and reflective type measurements, the optical sensor receives radiation that was emitted by the illumination unit into or onto the body part, and determines the received intensity and thereby the absorbance of the body part.

In pulse oximetry, use is commonly made of radiation at two different wavelengths such as to enable measurement at variable illumination strength, variable light path length and variable blood vessel diameter. Despite the illumination at two different wavelengths, the accuracy of such measurement systems is prone to artefacts such as movements and misalignment between the illumination unit and the sensor. As will be appreciated, since body parts have no regular shapes and the measurement is performed directly on body parts of a patient, a misalignment between the illumination unit and the sensor will often be present even if the mechanical setup is relatively stiff. Measured intensity of the radiation at the sensor side is dependent on the displacement and angle of incident and the exit angle of the radiation, and therefore a misalignment between the illumination unit and the sensor may introduce an error in the measured parameter. Misalignment can be reflected in x, y (perpendicular to source-sensor axis z) and phi, theta, psi (rotation around x, y, z axis respectively).

For example, the length of the optical path of radiation received by the sensor through the body part in a transmissive mode measurement is dependent on the angle of incident radiation received and the exit angle from the illumination source. Moreover, a fraction of the radiation received by the sensor may not have followed a straight or direct path between the illumination unit and the sensor. This fraction however will become larger in case of misalignment between the sensor and the illumination unit. This will also affect the accuracy of the results.

Another factor that determines the accuracy of the measurement systems, is that measurements often take place on living body parts. As will be appreciated, in a living body part, vibrations and small movements may continuously disturb the measurement process. These movements, caused by biological processes such as pulsation of the blood vessel, muscle contractions, a physical disorder of the patient or the like, may result in these motion artefacts that will influence the accuracy at which measurement may take place.

### Summary of the invention

It is an object of the present invention to obviate the above mentioned disadvantages of the prior art, and to provide a method and system for measuring a physiologic parameter of an organism which is less prone to misalignment and motion of a body part to be measured.

The above mentioned and other objects of the invention are achieved in that there is provided, in accordance with a first aspect, a method of measuring a parameter of a body, in particular a physiology parameter of an organism, such as a human or animal body, or a concentration of a substance in a contained volume, wherein said method is performed using a measurement system comprising an illumination unit and a sensor unit, the method comprising illuminating a body part of the body by providing electromagnetic radiation to an illumination area on the body part using the illumination unit, receiving by the sensor unit at least part of said radiation which has travelled via the body part, and analysing by a processing unit an output signal from the sensor unit using an analysis algorithm for determining a measurement value of said physiological parameter, wherein the step of illuminating of the body part is performed by emitting the radiation from a planar radiation emitting surface of the illumination unit for providing the radiation comprising a substantially planar homogeneous wave front in at least a part of the illumination area.

In accordance with the principles of the present invention, due to the fact that the electromagnetic radiation is emitted from a planar radiation emitting surface of the illumination unit, the radiation has a plane wave front in at least a part of the illumination area. In this part of the illumination area, the measurement results are far less (or utmost to a minimum extent) influenced by motion of the illumination unit or the sensor unit. In addition, influence of misalignment between the sensor unit and the illumination unit is diminished significantly. The use of a homogeneous plane wave front, the calculation of the physiological parameters to be determined becomes significantly less sensitive to alignment errors, placement errors, and mechanical, anatomical and lighting variations.

In this respect, the term homogeneous particularly refers to the intensity distribution across the illumination area. Although it is not necessary (although preferred) to have a perfectly flat distribution, local variations are preferably within limits. The term plane wavefront is known to the skilled person as meaning radiation wherein the intensity distribution across a plane transverse to the propagation direction is (more or less) uniform.

With respect to the homogeneity of the radiation, it is to be understood that preferably (but not necessarily) the light intensity to a distance of up to approximately two times a typical light source surface dimension (e.g. the length, width or radius of the used light source) from the planar radiation emitting surface in a plane that is perpendicular to the planar radiation emitting surface, is distributed equally within +/- 20% of candela of the average light intensity in a surface area of this plane having the same size as the planar radiation emitting surface. The skilled person may appreciate that this is dependent on the type of light source used. For a LED-array, dependent on the number of LED's per surface area, the radiation may not be sufficiently homogeneous having plane wave fronts at very close distance to the array.

In accordance with an embodiment of the present invention, the planar radiation emitting surface of the illumination unit is formed by at least one of: a light emitting surface of a homogeneous light source; or an output surface of an optical modifier unit, wherein the optical modifier unit is arranged for at least one of flattening the wave front or the homogeneity of the radiation.

As will be appreciated a suitable light source that comprises a planar light emitting surface providing light that is uniformly emitted from the surface will provide the desired radiation having a plane wave front. Such a light source may comprise at least one of a multispectral planar array, a plurality of light emitting diodes (LED's) arranged in a planar array, a multispectral planar array, a hyperspectral planar array, a bundle of optical fibers, a white colour light source, a broad band light source or a photo-organic film.

Alternatively, such radiation may also be obtained by using an arbitrary light source in combination with a suitable optical modifier unit. In that case, the modifier unit may cause the light received from the light source to be emitted again such that it is uniformly distributed across its surface. The optical modifier unit that may be suitable for flattening the wave front or the homogeneity of the radiation may comprise any one or more of a group comprising a diffuser, a lens, a lens array, a collimator or collimator array, a beam expander, a tunable filter such as a liquid crystal display matrix or any suitable combination of these optics.

The use of a planar radiation emitting surface of the illumination unit, enables a further improvement to the design of the system which allows for improving the accuracy of the measured results. This improvement become achievable when segments of the planar radiation emitting surface are individually operable, such that these may be switched on and off sequentially. In that case, the method may further comprise the steps of: sequentially lighting two or more segments of said planar radiation emitting surface; the processing unit determining from output signals of the sensor unit a received radiation intensity from each of the segments, and creating a measured attenuation map using the received radiation intensities determined from the illumination segments; and the processing unit comparing the measured attenuation map to a reference model of the measurement system for determining a difference, said reference model being at least one of an illumination model, a sensor model or an attenuation model, and estimating a misalignment contribution to the difference associated with geometric misalignment between the sensor unit and the illumination unit compared to a reference position. The term sensor model refers to a model of the sensitivities of the used sensor unit to variations of the angle of incidence, the position, aperture angle or any other relevant parameter.

The planar radiation emitting surface consisting of for example a plurality of light emitting diodes arranged in an array, enables independent lighting of segments of this surface. The same may be achieved with other types of planar light sources, or the combination of a light source with a suitable optical modifier unit. The independent switching of such segments enables to determine the light intensity received by the sensor in response to powering of each of the segments individually. This enables a range of opportunities to improve the accuracy of the system using an initialization algorithm.

An important improvement is achieved by creating a measured attenuation map using the received radiation intensities of each of the illumination segments determined. By comparing such a measured attenuation map to a reference attenuation model of the measurement system which is for example stored in a memory unit, it becomes possible to determine the difference between the measured attenuation map and an expected attenuation map, and to estimate a misalignment contribution which is associated with a geometric misalignment between the sensor unit and the illumination unit.

Moreover, a further possibility is that by sequentially powering of the different segments of the radiation emitting surface, it becomes possible to determine the exact angles and displacements between the sensing unit, the illumination unit and the tissue of the body part. As indicated, these parameters may be expressed in terms of x, y (perpendicular to source-sensor axis z) and phi, theta, psi (rotation around x, y, z axis respectively). This may advantageously be applied in a reflective and transmissive type measurement method. In a conventional type oximeter, for example, dependent on the type of measurement (reflective or transmissive) and on other factors such as whether or not the illumination unit provides collimated light, some of these parameters (x, y, z, phi, theta, psi) cannot be determined and as a result correction of the measurements is not possible. The measurement method in accordance with this embodiment of the present invention therefore provides a more reliable and accurate measurement of the parameter.

In accordance with the above mentioned embodiments a misalignment contribution to the difference between the measured attenuation map and the reference model is determined, which significantly improves the accuracy of the measured results. Further improvement of this method is possible by applying this technique as an iterative process. In this further embodiment, the method further comprises the steps of determining an anatomic contribution to the difference between the measured attenuation map and the reference attenuation model by comparing the misalignment contribution with the difference; comparing the anatomic contribution to an anatomic reference model of the body part for determining the presence or absence of a misfit between the anatomic contribution and the anatomic reference model; and in case a misfit is present, correcting the misalignment contribution associated with geometric misalignment based on the misfit.

In accordance with another embodiment of the present invention, the method further comprises a step of evaluating the attenuation map such as to identify locations wherein the overall attenuation of the radiation does not fit to the reference model corrected for misalignment. In case the fit of the misalignment correction is insufficient multispectral measurement gives additional information. Measurement is performed using multiple wavelengths of radiation such as to determine the anatomic contribution at the various locations across the planar radiation emitting surface dependent on the used wavelength. One of the wavelengths used for performing the measurements may be a wavelength that is mostly transparent to the substance or body part measured. Since both wavelengths are equally sensitive to misalignment but differ in sensitivity to anatomic variation, the misalignment and anatomic variation can be separated.

According to a second aspect of the invention, there is provided a measurement system for measuring a physiologic parameter of an organism, such as a human or animal body or a body substance in a contained volume, using a method according to the first aspect, the measurement system comprising an illumination unit for illuminating a body part of the organism by providing electromagnetic radiation to an illumination area on the body part, a sensor unit for receiving at least part of said radiation which has travelled through or against said body part, and a processing unit for analysing an output signal from the sensor unit using an analysis algorithm for determining a measurement value of said physiological parameter, wherein the illumination unit comprises a planar radiation emitting surface for providing the radiation comprising a substantially planar homogeneous wave front in at least a part of the illumination area.

The planar radiation emitting surface of the illumination unit may be formed by a light emitting surface of a homogeneous light source, or an output surface of an optical modifier unit arranged for at least one of flattening the wave front or the homogeneity of the radiation. In case a homogeneous light source is used, the homogeneous light source may be at least one of a plurality of light emitting diodes arranged in the planar array, a multispectral planar array, a hyper spectral planar array, a bundle of optical fibers, a white colour light source, a broad band light source, or a photo-organic film. In case the illumination unit comprises an optical modifier unit, the optical modifier unit may comprise at least one of a group comprising a diffuser, lens, lens array, a collimator, a beam expander, a tunable filter such as a liquid crystal display matrix, or any combination thereof.

It may be appreciated in accordance with a further embodiment, segments of the planar radiation emitting surface may be individually operable, and the measurement system may further comprise a control unit for sequentially lighting two or more segments of the light emitting surface. Where the radiation emitting surface comprises a plurality of light emitting diodes arranged in a flat array, the control unit may be arranged for sequentially powering two or more of the light emitting diodes. The array may consist of (n*m) light sources or LED's, where n is larger than or equal to 1 and m is larger than or equal to 1. An 1*m array of light sources may already be applied to resolve the largest of misalignment problems (e.g. in at least one dimension or relating to at least one angle) experienced with conventional light sources. This thereby reduces sensitivity to motion and anatomical variation in a subset of the translations and rotation directions, preferably the directions most sensitive to misalignment and anatomical variation.

The measurement system in accordance with this aspect of the invention may also comprise a memory unit for providing the processing unit with at least one of the reference illumination and attenuation model for the measurement system, and an anatomic reference model of the body part or substance to be measured.

In accordance with a third aspect, there is provided an optical physiology monitoring device, such as an oximeter, comprising the measurement system in accordance with the second aspect described herein above integrated in the device.

### Brief description of the drawings

The invention will further be illustrated by the description of some specific embodiments thereof, making reference to the attached drawings. The detailed description provides examples of possible implementations of the invention, but is not to be regarded as describing the only embodiments falling under its scope. The scope of the invention is defined in the appended claims, and the description is regarded as illustrative without being restrictive on the invention. In the drawings:
Figure 1 schematically illustrates the method of the present invention applied on a patient finger;
Figure 2 schematically illustrates a light source comprising a planar radiation emitting surface at two wavelengths for the use in a method according to the present invention;
Figure 3 schematically illustrates a measurement device for performing a method of the present invention;
Figure 4 schematically illustrates a misalignment between a planar light source and an optical sensor;
Figure 5 schematically illustrates a further illumination unit that may be applied in the method in accordance with the present invention;
Figure 6 schematically illustrates a further illumination unit that may be applied in a method in accordance with the present invention;
Figure 7 schematically illustrates a method in accordance with the present invention.

### Detailed description

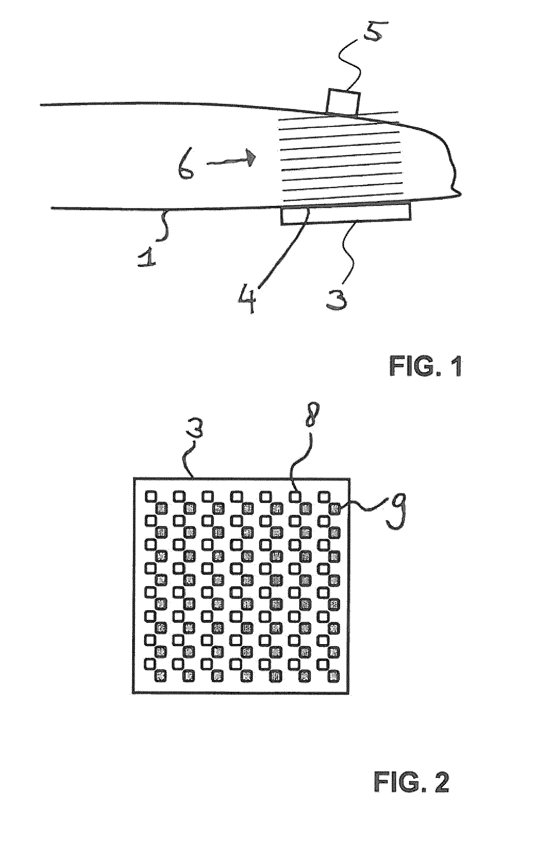

In figure 1, the method for measuring a physiologic parameter in accordance with the present invention is schematically illustrated. In figure 1, a patient's finger 1 is placed in between an illumination unit 3 and a sensor unit 5. The illumination unit 3 illuminates the finger 1 with electromagnetic radiation 6. This electromagnetic radiation is transmitted through the finger 1, and is received by the sensor unit 5. The illumination unit 3 and the sensor unit 5 are placed contiguous to the surface of the finger 1. As a result, as can be seen in figure 1, the sensor unit 5 and the illumination unit 3 will usually not be perfectly aligned. In the embodiment of figure 1, the measurement is performed in transmission, meaning that radiation provided by illumination unit 3 is transmitted by the finger 1 and is received by the sensor unit. The method may also be applied in a reflective type measurement method, wherein a diffusely and specular reflected fraction of radiation is received by a sensor unit. In a reflective type measurement, the illumination unit and the sensor unit are usually located at a same side of the body part.

The illumination unit 3 comprises a planar radiation emitting surface 4, from which the radiation 6 is emitted. The planar radiation emitting surface 4 is arranged for providing the radiation comprising a substantially planar homogeneous wave front to be illuminated on an illumination area on the finger 1. This is schematically illustrated by the horizontal lines 6 in figure 1, which indicate the homogeneous light intensity distribution at any cross section of the radiation.

It may be appreciated that figure 1 is a schematic illustration, and in reality internal reflections within a body part will have influenced the homogeneity and plane wave front character of the radiation, so that it will not reach the sensor unit 5 in the manner suggested in figure 1. This figure merely intends to explain that the illumination unit 3 provides homogeneous plane wave radiation. In which state of homogeneity the radiation will reach the sensor is dependent on many factors determined by the application.

The use of radiation having substantially a planar homogeneous wave front to a large extend reduces the sensitivity of the measurement method for motion artefacts, such as vibrations of the body part 1, undesired muscle induced motions, and biological processes within body part 1. Moreover, as a result of using plane wave radiation, the effect of any misalignment that may be present between illumination unit 3 and sensor unit 5 on the accuracy of the measurement method may be eliminated or at least minimized.

Figure 2 schematically illustrates a light source that may be used in an illumination unit 3 for providing radiation comprising a substantially planar homogeneous wave front. In particular, the light source comprises a first array of light emitting diodes 8 of a first wavelength, and a second array of light emitting diodes of a second wavelength. Although each of the LED's (such as LED 8 or 9) may be individually operable (as will be explained later), for performing the measurement of the parameter at least all the light emitting diodes of one of the wavelengths (e.g. the light emitting diodes 8 of the first array) will be lit. Prior to performing the measurement itself, or one or more times in between individual measurements, an initialization method may be applied for determining any remaining contribution of misalignment between a sensor unit 5 and the illumination unit 3.

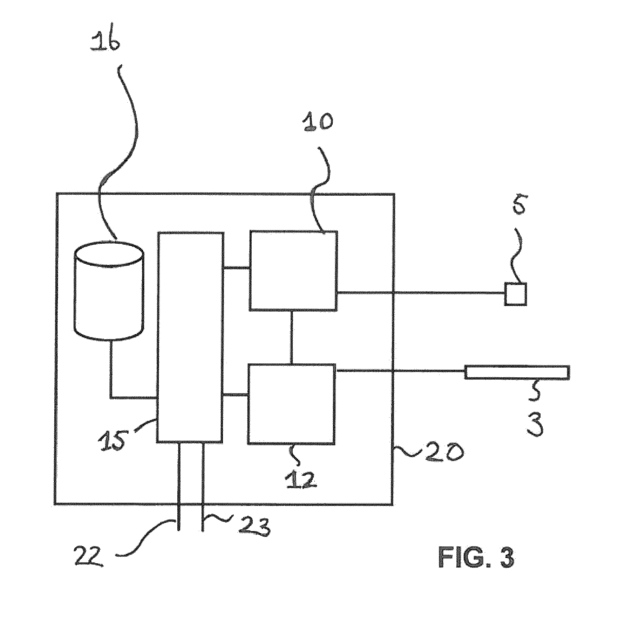

A parameter monitoring device for performing the measurement method of the present invention is schematically illustrated in figure 3. The device comprises the sensor unit 5 and the illumination unit 3. Moreover, the sensor unit 5 is connected to a processor unit 10 and the processor unit 10 cooperates with a control unit 12 which, in turn, also controls lighting of the segments of the illumination unit 3. Relevant data may be transmitted or received using a communication unit 15 through input port 22 and output port 23 of the device. Input port 22 and output port 23 of the device allow the device to be connected to a further processing apparatus, such as a computer system. Input and output ports may also be embodied through wireless communication. Data may also be stored in a memory unit 16 within the device 20.

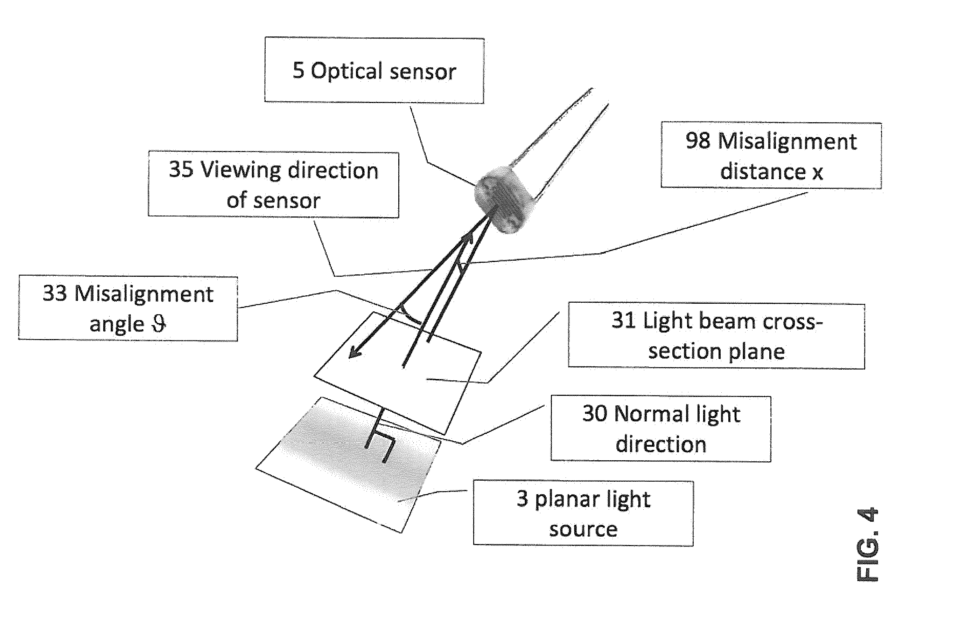

Figure 4 schematically illustrates a misalignment between an optical sensor 5 and a planar light source 3. The planar light source 3 emits radiation which propagates in the normal light direction 30. Light beam cross-section plane 31 schematically illustrates the alignment of the plane wave front. Optical sensor 5 is not exactly aligned with the planar light source 3, and has a viewing direction 35 which is at an angle 33 with the propagation direction 30 of the planar wave front. Due to the fact that the radiation emitted by the illumination unit 3 comprises planar homogeneous wave fronts, a misalignment angle between the viewing direction 35 of the sensor and the propagation direction 30 of the radiation will have only a minimal effect on the accuracy of the measurement. As will be appreciated, this will be different in case an arbitrary or conventional light source is used such as a regular light emitting diode from which the emitted light propagates with curved wave fronts. In that case, the viewing direction 35 of the sensor 5 causes the sensor 5 to receive less radiation per surface area in case the angle 33 and misalignment distance 98 between the viewing direction 35 and the propagation direction 30 increases. Moreover, variable misalignment between the sensor 5 and the planar light source 3 also causes the optical path of the radiation from the light source to the optical sensor to increase or decrease.

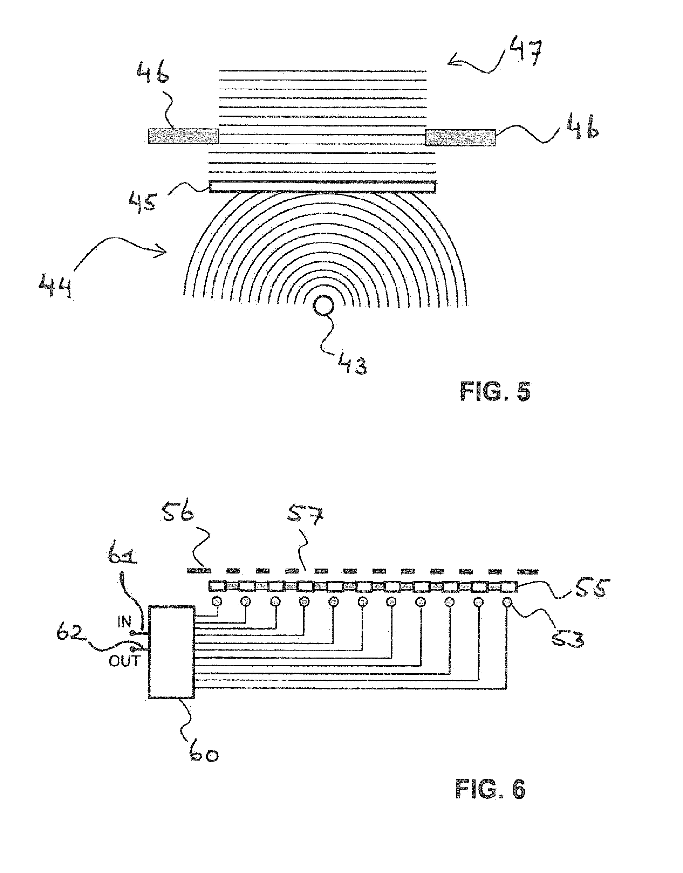

Figure 5 illustrates a further embodiment of an illumination unit in accordance with the present invention. In the illumination unit illustrated in figure 5, a regular single point light source 43 emits radiation 44 which is transmitted to a positive lens 45. The radiation transmitted by light source 43 is not homogeneous plane wave radiation. The light source 43 is placed at focal distance from the lens 45, and as a result, the radiation leaving the lens 45 is collimated and comprises plane wave fronts behind the lens 45. In order to remove any edge effects near the edges of the lens, a diaphragm 46 partly shields the emitted radiation from the lens 45, resulting in clear plane waves 47 at the front side of the illumination unit. As will be appreciated, the illumination unit may comprise a plurality of light sources such as light source 43, each comprising a lens.

In figure 6, such an embodiment of an illumination unit is schematically indicated. In figure 6, a plurality of points shaped light sources 53 such as light emitting diodes, are arranged behind a lens array 55. The lens array comprises a plurality of small lenses creating plane wave fronts at the opposite side thereof. A plane diaphragm array 56 removes any undesired edge effects, resulting in neat and clean plane wave fronts at the front side of the illumination unit. It may be appreciated that plane wave fronts can already be obtained without the use of a lens array or collimator.

Control unit 60 with input and output ports 61 and 62 respectively, allows for individual operation of each or groups of the light sources 53 of the illumination unit. This is used for enabling an initialization method, for which any effects of geometric misalignment between the sensor unit and the illumination unit may be eliminated.

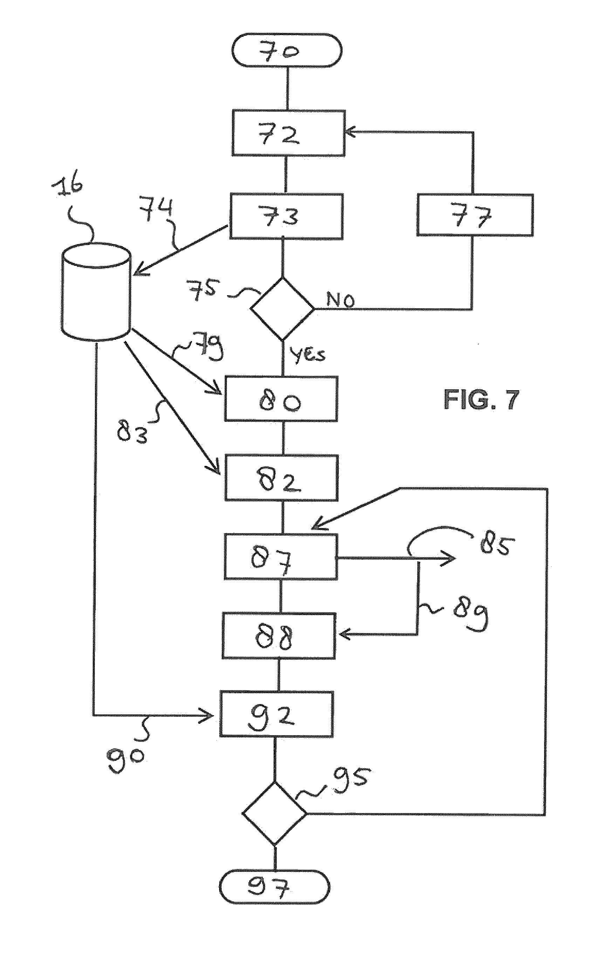

Such an initialization method is schematically illustrated in figure 7. Figure 7 starts with step 70 wherein the first light emitting diode of the array is selected by the control unit. In step 72, a control unit powers the selected light emitting diode, and the light intensity from the radiation provided by the first light emitting diode is received in step 73 by the sensor unit. The processor unit which determines the light intensity received by the sensor unit stores this data in the memory unit 16. In step 75, the control unit determines whether at least the minimum required number of the segments of the array have been lit sequentially. This is not the case, in step 77 a next light emitting diode is selected by the control unit which is again temporarily powered in step 72. This process continues until at least the minimum required number of the light emitting diodes have been lit by the control unit, and the corresponding received intensities 74 have been stored in the memory unit 16. The minimum required number of segments to light is determined by the number and/or nature of parameters to correct the measurement results for.

In case the control unit determines in step 75 that all the necessary light emitting diodes have already been lit, the initialization process continues in step 80, wherein from the received radiation intensity data 79, an attenuation map is created by the processor unit. In step 82, the processor unit retrieves from the memory unit 16 a reference model of the measurement system from which a difference between the attenuation map created in step 80 and an expected attenuation map can be obtained. The reference model used by the processing unit and stored in the memory unit 16 may be an illumination model or attenuation model or sensor model or any other model that allows for the comparison to be made. From the difference determined by the processing unit between the attenuation map created from the received radiation intensities 79 and the reference model, the processing unit estimates a misalignment contribution to the difference associated with geometric misalignment between the sensor unit and the illumination unit compared to a reference position. The processor unit uses a mathematical algorithm from which the contribution of translations and rotations of the sensor relative to the illumination unit can be calculated, using the difference as input. This calculation may be performed independent of anatomical variation in the illuminated volume of the body part 1, especially when using multiple wavelengths. The estimation of the misalignment contribution to the difference is performed in step 87, and the output, being the misalignment contribution or alternatively the amount of misalignment in terms of rotation and translation, is provided as data schematically indicated in figure 7 as output 85.

As will be appreciated by the skilled person, this data may be stored as well in memory unit 16 and used to correct any measurement taken after the initialization method, once the real measurements take place. The use of this misalignment data or misalignment contribution significantly enhances the accuracy of the measurement method performed. Although referred to as 'initialisation method', it must be understood that this method may be applied multiple times during measurement, e.g. every second or every 20 seconds. This may be advantageous in view of movement of the body part. The misalignment contribution however may also be used in an iterative process and transmitted back as input 89 to step 88. In step 88, the anatomic contribution to the difference determined in step 82 between the measured attenuation map and the reference model may be determined by comparing the misalignment contribution 89 with the difference itself. To this end, the misalignment contribution 89 (also output 85) may be compensated for the difference determined in step 82 by e.g. subtraction. Alternatively, it is also possible to divide both parameters by each other, to determine whether or not there is a misfit or mismatch between the difference and the misalignment contribution. This is in step 92 compared to an anatomic reference model retrieved from memory unit 16 (input 90). By making this last comparison, the processing unit is able to determine whether the identified anatomic contribution obtained by comparing the misalignment contribution and the difference in step 88, is fully associated with the anatomy of the body part, or whether there is a misfit that may give rise to a further correction of the misalignment contribution. In step 95, it is determined whether a misfit is present that gives rise to a further correction of the misalignment contribution. If this is the case, this information is provided as data to step 87 and the processor is iterated until no further improvement may be obtained. In case no misfit is present, the initialization method is terminated in step 97, and the method of the present invention continues by performing a measurement of the desired parameter. The process of misalignment determination and anatomical compensation can be repeated for each measurement which may take place between many times a minute.

The present invention has been described in terms of some specific embodiments thereof. It will be appreciated that the embodiments shown in the drawings and described here and above are intended for illustrative purposes only, and are not by any manner or means intended to be restrictive on the invention. The context of the invention discussed here is merely restricted by the scope of the appended claims.

## Claims

1. Method of measuring a parameter of a body, in particular a physiologic parameter of an organism, such as a human or animal body, or a concentration of a substance in a contained volume, wherein said method is performed using a measurement system comprising an illumination unit and a sensor unit, the method comprising illuminating a body part of the body by providing electromagnetic radiation to an illumination area on the body part using the illumination unit, receiving by the sensor unit at least part of said radiation which has travelled via the body part, and analysing by a processing unit an output signal from the sensor unit using an analysis algorithm for determining a measurement value of said physiological parameter, wherein the step of illuminating of the body part is performed by emitting the radiation from a planar radiation emitting surface of the illumination unit for providing the radiation comprising a substantially planar homogeneous wave front in at least a part of the illumination area.

2. Method according to claim 1, wherein the planar radiation emitting surface of the illumination unit is formed by at least one of:
a light emitting surface of a homogeneous light source; or
an output surface of an optical modifier unit, wherein the optical modifier unit is arranged for at least one of flattening the wave front or the homogeneity of the radiation.

3. Method according to claim 1 or 2, wherein the radiation provided by the illumination unit is collimated radiation.

4. Method according to any of the previous claims, wherein segments of the planar radiation emitting surface are individually operable, and wherein the method further comprises the steps of:
sequentially lighting two or more segments of said planar radiation emitting surface;
the processing unit determining from output signals of the sensor unit a received radiation intensity from each of the segments, and creating a measured attenuation map using the received radiation intensities determined; and
the processing unit comparing the measured attenuation map to a reference model of the measurement system for determining a difference, said reference model being at least one of an illumination model, a sensor model or an attenuation model, and estimating a misalignment contribution to the difference associated with geometric misalignment between the sensor unit and the illumination unit compared to a reference position.

5. Method according to claim 4, wherein the misalignment contribution is determined in terms of at least one of a relative displacement or a relative rotation between the sensor unit and the illumination unit in relation to the reference position.

6. Method according to any of claims 4 or 5, further comprising a step of:
determining an anatomic contribution to the difference between the measured attenuation map and the reference attenuation model by comparing the misalignment contribution with the difference;
comparing the anatomic contribution to an anatomic reference model of the body part for determining the presence or absence of a misfit between the anatomic contribution and the anatomic reference model; and
in case a misfit is present, correcting the misalignment contribution associated with geometric misalignment based on the misfit.

7. Method according to any of the claims 4-6, wherein the step of sequentially lighting is performed with radiation at two or more wavelengths to determine attenuation at each wavelength for a plurality of locations across the planar radiation emitting surface.

8. Method according to any of the previous claims, wherein segments of the planar radiation emitting surface are individually operable, and wherein the method further comprises the steps of:
sequentially lighting two or more segments of said planar radiation emitting surface;
the processing unit determining from output signals of the sensor unit an angle or displacement between the sensor unit and an outer surface of the body part.

9. Measurement system for measuring a physiologic parameter of an organism, such as a human or animal body, using a method in accordance with any of the claims 1-8, the measurement system comprising an illumination unit for illuminating a body part of the organism by providing electromagnetic radiation to an illumination area on the body part, a sensor unit for receiving at least part of said radiation which has travelled through or against said body part, and a processing unit for analysing an output signal from the sensor unit using an analysis algorithm for determining a measurement value of said physiological parameter, wherein the illumination unit comprises a planar radiation emitting surface for providing the radiation comprising a substantially planar homogeneous wave front in at least a part of the illumination area.

10. Measurement system according to claim 9, wherein the planar radiation emitting surface of the illumination unit is formed by at least one of:
a light emitting surface of a homogeneous light source; or
an output surface of an optical modifier unit, wherein the optical modifier unit is arranged for at least one of flattening the wave front or the homogeneity of the radiation.

11. Measurement system according to claim 10, wherein
the homogeneous light source comprises at least one of a multispectral planar array, a plurality of light emitting diodes arranged in a planar array, a hyperspectral planar array, a bundle of optical fibres, a white colour light source, a broad band light source, a linear array of (1*n) light sources or a photo-organic film; or
wherein the optical modifier unit comprises at least one of a group comprising a diffuser, a lens, lens array, collimator, beam expander a tunable filter such as a liquid crystal display matrix, or a combination thereof.

12. Measurement system according to any of the claims 9-11, wherein segments of the planar radiation emitting surface are individually operable, and wherein said measurement system further comprises a control unit for sequentially lighting two or more segments of said light emitting surface.

13. Measurement system in accordance with claim 11 and 12, wherein the illumination unit comprises said homogeneous light source comprising a plurality of light emitting diodes arranged in a flat array, wherein the control unit is arranged for sequentially powering two or more of said light emitting diodes.

14. Measurement system in accordance with any of the claims 9-13, further comprising a memory unit for providing said processing unit with at least one of a reference illumination and attenuation model for the measurement system, a sensor model, and an anatomic reference model of the body part.

15. Optical physiology monitoring device, such as an oximeter, comprising a measurement system according to any of the claims 9-14, integrated in the device.
